Europäisches Patentamt

European Patent Office

Office européen des brevets

(11) Numéro de publication: **0 363 249**
A1

## DEMANDE DE BREVET EUROPEEN

(21) Numéro de dépôt: **89402608.7**

(22) Date de dépôt: **22.09.89**

(51) Int. Cl.5: **A61B 6/08** , **A61N 5/10**

(30) Priorité: **04.10.88 FR 8812973**

(43) Date de publication de la demande:
**11.04.90 Bulletin 90/15**

(84) Etats contractants désignés:
**CH DE GB LI NL SE**

(71) Demandeur: **CGR MEV**
**551, rue de la minière, BP 34**
**F-78530 Buc(FR)**

(72) Inventeur: **Trotel, Jacques**
**CABINET BALLOT-SCHMIDT 7, Rue Le Sueur**
**F-75116 Paris(FR)**

(74) Mandataire: **Ballot, Paul Denis Jacques et al**
**Cabinet Ballot-Schmit 7, rue le Sueur**
**F-75116 Paris(FR)**

(54) **Système et procédé de mesure et/ou de vérification de la position d'un patient dans un équipement de radiothérapie.**

(57) L'invention concerne les équipements et installa-tions de radiothérapie.

L'invention réside dans le fait que l'on mesure ou vérifie la position précise d'un patient à l'aide d'un dispositif (37, 38) monté sur le bras mobile (34) d'un statif (30) à mouvement isocentrique. Ce dispo-sitif comprend un système de balayage (37) d'un faisceau lumineux dont la position de la source cor-respond à celle de la source de rayonnement (36) et un système de détection optique (38) du point d'im-pact du faisceau lumineux sur le patient, ces deux systèmes permettant de déterminer à l'aide d'un système informatique (32) la position du point d'im-pact. L'invention est applicable à la radiothérapie.

EP 0 363 249 A1

# SYSTEME ET PROCEDE DE MESURE ET/OU DE VERIFICATION DE LA POSITION D'UN PATIENT DANS UN EQUIPEMENT DE RADIOTHERAPIE

L'invention concerne la radiothérapie et, plus particulièrement dans un équipement de radiothérapie, un système et un procédé de mesure et de vérification de la position d'un patient.

Un traitement de radiothérapie est décidé en fonction d'un ensemble d'informations de type diagnostique comprenant essentiellement la nature, l'étendue et la localisation de la tumeur et des informations sur l'état général du patient. Le traitement est défini par un plan de traitement qui est établi de façon à délivrer une dose prescrite de rayonnement au volume tumoral et la dose la plus faible possible aux tissus ne faisant pas partie du volume tumoral. A cet effet, le plan de traitement définit la nature, l'intensité, l'orientation et l'étendue des faisceaux qui seront utilisés pour irradier le patient et il est établi à partir de données géométriques fournies par un simulateur et des résultats de calculs de doses fournis par un système informatique spécialisé.

Le simulateur de radiothérapie est constitué essentiellement d'un appareil de radiologie par rayons X qui reproduit la géométrie de l'appareil de radiothérapie mais qui utilise une source de rayons X de type diagnostique, c'est-à-dire de faible énergie comparée à celle de la source de rayonnement haute énergie de l'appareil de radiothérapie. Cet appareil de radiologie est associé à un système d'imagerie permettant de visualiser les organes et tissus traversés par le faisceau de rayons X.

Pour traiter la tumeur, il est nécessaire de connaître de manière précise sa position par rapport à la source de rayonnement. Pour cela, le simulateur comprend divers moyens optiques pour repérer la position relative du patient et de l'appareil de radiologie. L'un de ces moyens est un télémètre qui a pour but de mesurer la distance entre la source de rayons X de radiothérapie et le point d'entrée de l'axe du faisceau de rayons X dans le corps du patient. Cette distance est appelée distance source-peau.

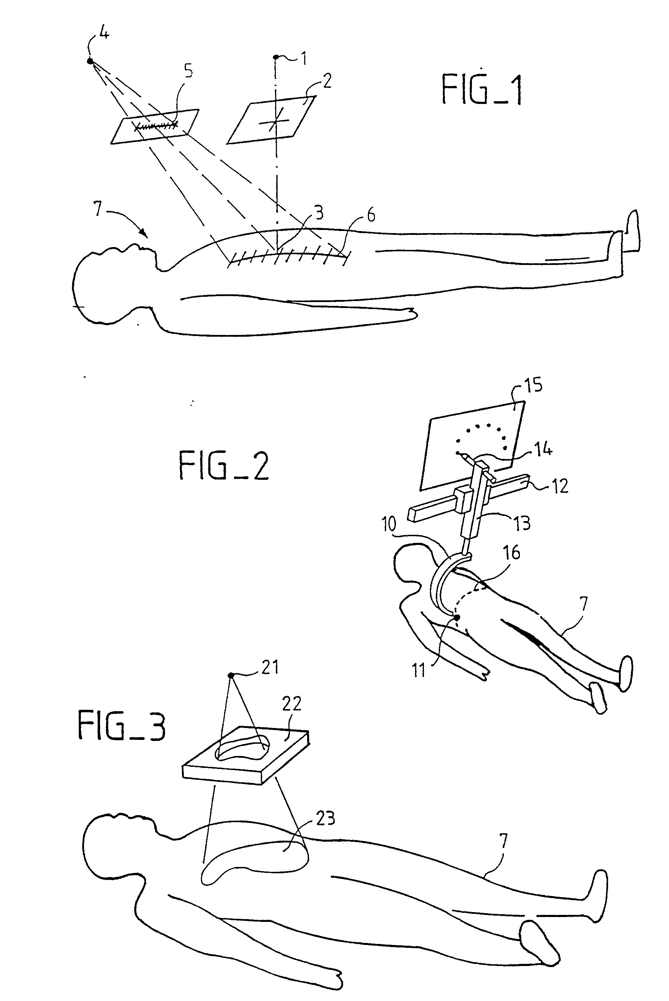

Comme le montre la figure 1, un télémètre du type utilisé dans un simulateur de radiothérapie comprend une première source lumineuse 1 qui projette sur un patient 7 l'ombre d'une croix 2. La source lumineuse 1 est confondue avec la source de rayons X et le point d'intersection des deux branches de la croix 2 est situé sur l'axe du faisceau X. Le point 3, ombre projetée sur la peau du patient du point d'intersection des deux branches de la croix 2, est donc confondu avec le point d'entrée de l'axe du faisceau X dans le corps du patient 7.

Le télémètre comprend également une deuxième source lumineuse 4 qui projette sur le patient 7 l'ombre d'une échelle graduée 5. La source 4 et l'axe de l'échelle graduée 5 sont dans le même plan que l'axe du faisceau de rayons X et il en résulte que l'axe de l'ombre 6 de l'échelle graduée 5 passe par le point 3.

Le repère de l'ombre de l'échelle graduée 5 qui se trouve en coïncidence avec le point 3 dépend de la distance entre la source 1 et le point 3 de sorte que l'observation de ce repère en coïncidence avec le point 3 fournit donc une mesure de la distance source-peau.

Le système informatique de calcul de dose utilise comme données, d'une part, la géométrie et la nature des faisceaux et, d'autre part, des informations sur l'anatomie du patient dans la région exposée au rayonnement.

Les informations anatomiques sont en général présentées sous la forme d'images qui représentent la densité des tissus du patient dans des sections coronales du patient. Les images de ces sections sont fournies par plusieurs moyens :
- l'appareil de radiologie du simulateur qui fournit des vues sous diverses incidences;
- un tomodensitomètre qui fournit des informations sur la nature des tissus,
- un conformateur qui fournit des relevés du contour externe du patient dans différents plans de sections.

La figure 2 permet de comprendre en quoi consiste un conformateur. Il comprend un palpeur 10 dont la pointe 11 ne peut se déplacer que dans un plan défini par deux rails de déplacement 12 et 13. Ce plan est amené en coïncidence avec le plan de la section dont on veut relever le contour externe. Le palpeur 10 est muni, à l'extrémité opposée à sa pointe 11, d'un crayon 14 qui est prévu pour tracer des points sur une feuille de papier 15 placée dans un plan parallèle au plan de section. On comprend que, par construction, le mouvement du crayon 14 se déduit par translation de celui de la pointe 11. Ainsi pour relever un contour externe 16, la pointe 11 du palpeur 10 est amenée en contact avec quelques positions significatives sur la peau du patient 7 et, pour chacune de ces positions, un point est tracé par le crayon 14 sur la feuille 15.

Les différentes opérations qui viennent d'être succinctement décrites permettent d'établir le plan de traitement radiothérapique. Pour le mettre en oeuvre, il faut définir la position relative des différents faisceaux de rayons X prévus par le traitement par rapport au corps du patient. Ceci est obtenu en traçant des repères au marqueur indélébile sur la peau du patient de manière à indiquer le contour de chacun des faisceaux de rayons X. Pour permettre ces différents tracés, les faisceaux de rayons X sont visualisés par des moyens optiques appelés délinéateurs.

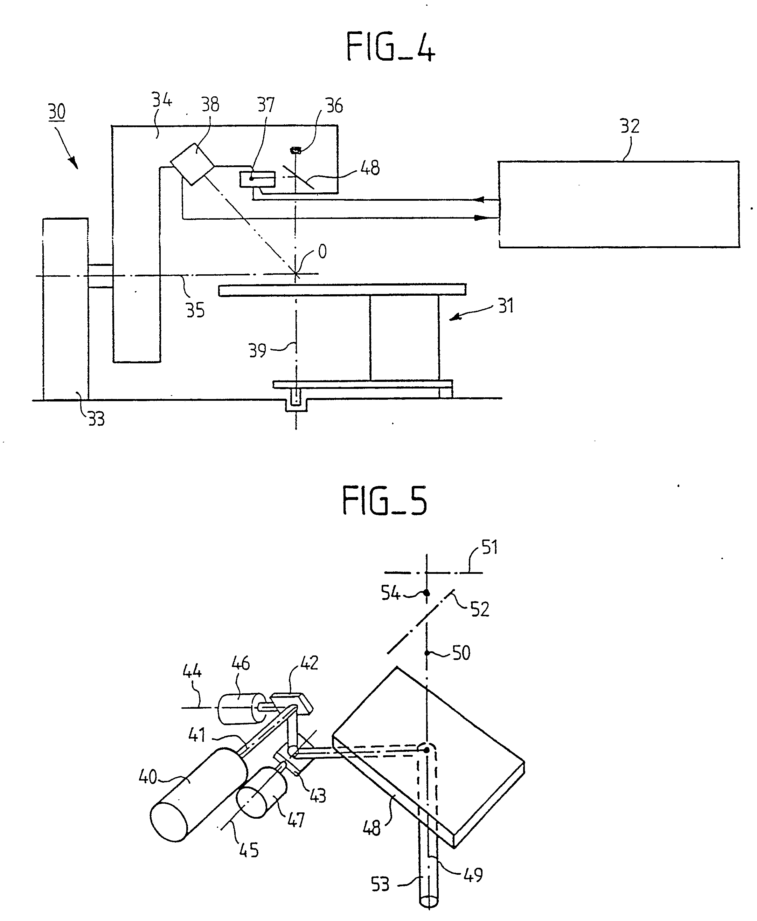

Un délinéateur (figure 3) consiste en une source lumineuse 21, placée au point origine du faisceau de rayons X, qui projette sur la peau du patient l'ombre d'un diaphragme 22. La forme du diaphragme est telle que sa projection 23 sur la peau du patient représente la trace de l'entrée du faisceau de rayons X dans le patient et est utilisée pour tracer les repères sur la peau du patient.

Le diaphragme 22 est constitué habituellement d'un ensemble de plaques mobiles à bords rectilignes lorsque le faisceau est rectangulaire ou d'une plaque qui est découpée spécialement lorsque le faisceau est d'une forme plus complexe.

Lorsque les opérations qui viennent d'être décrites sont terminées, le patient peut alors être traité dans une installation de radiothérapie, la première opération du traitement consistant à le placer dans une position qui correspond le mieux possible à celle qu'il avait sur le simulateur. A cet effet, cette installation de radiothérapie comporte divers moyens optiques qui correspondent à ceux du simulateur, en particulier le télémètre.

Avant l'irradiation du patient, la position des faisceaux de thérapie par rapport au patient est vérifiée à l'aide d'un délinéateur identique à celui du simulateur de manière à s'assurer que la trace lumineuse ainsi obtenue correspond aux marques tracées sur la peau du patient.

Les inconvénients des systèmes de simulation de l'art antérieur sont principalement les suivants.

La mesure de la distance source-peau nécessite la présence d'un opérateur à proximité du patient pour observer les traces lumineuses projetées par le télémètre. Il en résulte une perte de temps et des risques d'erreur. Mais surtout, la mesure ne peut être faite pendant l'irradiation car l'opérateur ne peut rester près du patient de sorte que des mouvements involontaires du patient ne sont pas détectés et peuvent ainsi conduire à une irradiation qui n'est pas conforme au plan de traitement.

Le relévé du contour externe d'une section du patient au moyen du conformateur est une opération qui est lente, manque de précision et nécessite la présence d'un opérateur près du patient. Elle ne peut donc pas être effectuée pendant l'irradiation, ce qui empêche d'utiliser cette mesure comme moyen supplémentaire de vérification de la conformité du traitement au plan de traitement.

L'opération de délinéation optique n'est rapide que lorsque le faisceau a une section rectangulaire. Dans des cas plus complexes, il faut réaliser des diaphragmes spécifiques dont la confection est lente.

Le but de la présente invention est donc de réaliser un système de mesure et de vérification de la position d'un patient qui ne présente pas les inconvénients précités des systèmes de l'art antérieur et qui réalisent les fonctions du télémètre, du conformateur et du délinéateur décrites ci-dessus dans le préambule.

L'invention se rapporte à un système de mesure et/ou de vérification de la position d'un patient dans un équipement de radiothérapie qui comprend un statif à mouvement isocentrique, une table sur laquelle est disposé le patient et une source de rayonnement qui peut être à basse énergie pour des opérations de simulation ou à haute énergie pour des opérations de radiothérapie, caractérisé en ce qu'il comprend un dispositif de balayage d'un faisceau lumineux suivant au moins une direction de manière à pouvoir balayer au moins une partie de la surface du corps du patient et un dispositif de commande des positions du faisceau lumineux de manière à déplacer le faisceau lumineux selon un tracé déterminé sur la surface du corps du patient, ledit tracé délimitant la surface d'impact du faisceau de rayons haute énergie.

Le système comprend en outre un dispositif de détection de l'impact du faisceau lumineux sur le corps du patient et un dispositif de calcul connecté au dispositif de commande de position du faisceau lumineux et au dispositif de détection pour calculer la position de l'impact du faisceau lumineux sur le corps du patient de manière à pouvoir déterminer, d'une part, la distance entre la source de rayons haute énergie et la surface du corps du patient et, d'autre part, le contour extérieur d'une section du corps du patient.

L'invention se rapporte également à un procédé de mesure et/ou de vérification de la position d'un patient dans un équipement de radiothérapie qui comprend un statif à mouvement isocentrique, une table sur laquelle est disposé le patient et une source de rayonnement qui peut être à basse énergie pour des opérations de simulation ou à haute énergie pour des opérations de radiothérapie, ladite source de rayonnement étant portée par le statif, caractérisé en ce qu'il comprend les opérations suivantes :

a) un balayage d'au moins une partie de la surface du corps du patient par un faisceau lumineux dont la source est confondue avec la source de rayonnement de manière à obtenir des points d'impact déterminés dudit faisceau sur la surface du corps du patient,

b) une détection de ces points d'impact par l'enregistrement d'une image appropriée,

c) un calcul de la position de ces points d'impact à partir de la connaissance de la position de la source et de la position sur l'image de ces points d'impact.

d) l'enregistrement des positions de ces points d'impact en relation avec les positions correspondantes du faisceau lumineux qui leur a donné naissance.

Le procédé comprend en outre les opérations suivantes :

e) un balayage du corps du patient par le faisceau lumineux à l'aide des informations de positions enregistrées du faisceau lumineux pour obtenir les points d'impact dont les positions ont été enregistrées,

f) la mesure de la position réelle de ces points d'impact en mettant en oeuvre les opérations a), b), c), et d).

g) la comparaison des positions réelles des points d'impact avec les positions enregistrées et,

h) le déplacement du corps du patient pour que les positions réelles coïncident avec les positions enregistrées.

D'autres caractéristiques et avantages de la présente invention apparaîtront à la lecture de la description suivante d'un exemple particulier de réalisation, ladite description étant faite en relation avec les dessins joints dans lesquels :

- la figure 1 est un schéma qui permet de comprendre le principe de la mesure de la distance source-peau,

- la figure 2 est un schéma qui permet de comprendre le principe de fonctionnement d'un conformateur de contour externe d'un patient,

- la figure 3 est un schéma qui permet de comprendre le principe de fonctionnement d'un délinéateur de trace de faisceau sur un patient,

- la figure 4 est une vue schématique d'un système de mesure et de vérification de la position d'un patient dans un équipement de radiothérapie selon l'invention, et

- la figure 5 est une vue en perspective cavalière d'un dispositif de balayage optique qui peut être mis en oeuvre pour réaliser le système selon l'invention.

Les figures 1 à 3 qui ont été utilisées pour expliquer le principe de fonctionnement du télémètre, du conformateur et du délinéateur de l'art antérieur ne sont pas décrites à nouveau.

La figure 4 représente un équipement qui peut être à la fois un appareil de radiothérapie et un système de mesure et de vérification de la position d'un patient. Cet équipement comprend un statif 30, une table de support 31 d'un patient et un système informatique 32. Le statif 30 est du type à mouvement isocentrique et peut être réalisé de différentes manières qui sont connues par ailleurs. Il comprend par exemple un pied 33 qui supporte un bras 34 en forme de L; ce bras peut tourner autour d'un axe horizontal 35. La partie du bras au-dessus de la table 31 sert de support à un certain nombre d'éléments qui sont une source de rayonnement X ou de rayonnement ionisant 36, un dispositif de balayage lumineux 37 et un récepteur d'image 38. Le dispositif de balayage 37 et le récepteur d'image 38 sont connectés à un système informatique 32. La table 31 est prévue pour être déplacée suivant différents axes de manière à ajuster la position du patient par rapport au statif. Par exemple, les mouvements que peut effectuer la table sont un mouvement de translation verticale, un mouvement de translation horizontale et un mouvement de rotation autour d'un axe vertical 39 qui coïncide avec l'axe de rayonnement de la source 36 lorsque cette dernière est située dans le plan de la figure 4. Il est à noter que sur la figure 4, l'isocentre est référencé par la lettre O.

Dans une installation de radiothérapie, la source de rayonnement 36 fournit un rayonnement de haute énergie. Dans un simulateur de radiothérapie, la source de rayonnement 36 fournit un rayonnement X de faible énergie et est prévue pour coopérer avec un récepteur, non représenté sur la figure 4, situé de l'autre côté du patient par rapport à la source.

La figure 5 représente un exemple de réalisation du dispositif de balayage lumineux 37. Il comprend une source lumineuse 40, de préférence un laser, qui émet un faisceau étroit de lumière visible 41. Il comprend également deux miroirs tournants 42 et 43 qui oscillent respectivement autour d'axes 44 et 45, l'un 44 perpendiculaire à l'axe du faisceau lumineux 41 qu'il reçoit directement et l'autre perpendiculaire au premier et parallèle à l'axe du faisceau 41. L'oscillation de ces miroirs 42 et 43 est obtenue respectivement par des déflecteurs galvanométriques ou des moteurs 46 et 47 qui sont commandés par le système informatique 32.

Le faisceau lumineux 41 est réfléchi par ces deux miroirs 42 et 43 pour être envoyé sur un troisième miroir fixe 48 qui le réfléchit en direction du corps du patient suivant un axe 49 en position de repos des miroirs 42 et 43. Cet axe 49 coïncide avec l'axe central de rayonnement 50 de la source de rayonnement 36. Le miroir 48 est transparent au rayonnement utilisé.

Les mouvements d'oscillations du miroir 42 produisent une oscillation du faisceau 41 autour d'un axe 51 qui se déduit de l'axe 44 par une première symétrie par rapport au miroir 43 puis par une deuxième symétrie par rapport au miroir 48.

Les mouvements d'oscillation du miroir 43 produisent une oscillation du faisceau 41 autour d'un axe 52 qui se déduit de l'axe 45 par une symétrie par rapport au miroir 48.

Si les miroirs 42 et 43 sont très proches l'un de l'autre, il en sera de même des axes 51 et 52 et les mouvements combinés des miroirs 42 et 43 produiront sensiblement un mouvement d'oscillation du faisceau 53 autour d'un point 54 situé sur la perpendiculaire commune aux axes 51 et 52 et à égale distance de ces deux axes.

De préférence, les miroirs 42, 43 et 48 sont disposés de telle sorte que le point 54 est confondu avec le point source de rayons X dans le cas du simulateur ou de rayons de haute énergie, rayons X ou autres, dans le cas d'un appareil de radiothérapie.

De préférence également, les axes 51 et 52 sont perpendiculaires entre eux et perpendiculaires à l'axe 50 du faisceau de rayons X (ou de rayons de haute énergie) et l'un des axes 51 ou 52 est parallèle à l'axe de rotation 35 du bras 34 (figure 4).

Le dispositif de balayage 37 permet de faire décrire au faisceau lumineux 41, en fonction des signaux appliqués aux déflecteurs 46 et 47, la surface externe du faisceau de rayonnement X ou haute énergie. La trace du faisceau 53 sur la peau du patient représente alors les limites de la trace du faisceau de rayonnement X ou de haute énergie à son entrée dans le patient. On peut ainsi réaliser la fonction du délinéateur sans avoir à réaliser un diaphragme comme dans l'art antérieur.

Lors des opérations de simulation, le balayage du faisceau 53 est commandé par l'opérateur pour obtenir la trace repérée sur le corps du patient; les informations de commande sont enregistrées sur un support mémoire pour être ensuite utilisées sur le dispositif de balayage de l'appareil de radiothérapie et ainsi obtenir la même trace sur le patient que le marquage indélébile en déplaçant ce dernier pour qu'il en soit ainsi.

Le dispositif de balayage qui a été décrit à titre d'exemple comprend deux miroirs oscillants mais on peut réaliser le même balayage avec un seul miroir qui serait animé de mouvements suivant deux axes perpendiculaires. Afin de réaliser les fonctions du conformateur et du télémètre de l'art antérieur, l'invention prévoit d'associer au dispositif de balayage 37 le récepteur d'image 38. Ce récepteur d'image 38 est prévu pour fournir une image plane de toute la région de l'espace où peut se trouver le point d'impact du faisceau lumineux 53 sur la peau du patient. A l'aide de cette image du point d'impact et des données géométriques d'orientation et de position du faisceau 53 ainsi que du récepteur, il est possible de déterminer la position dans l'espace du point d'impact du faisceau lumineux 53 sur le corps du patient.

De tels dispositifs sont connus et sont par exemple décrits dans le brevet US 4593967.

En déplaçant le faisceau lumineux sur la peau du patient, on obtient un relevé en trois dimensions de la surface externe du patient qui peut être atteinte par le faisceau lumineux pour une certaine position angulaire du bras 34. Avec différentes positions angulaires du bras 34, il est possible d'avoir pratiquement un relevé de la surface externe du patient qui présente de l'intérêt pour le traitement de radiothérapie.

Dans un tel relevé, la mesure de la distance source-peau n'est qu'un cas particulier correspondant au faisceau lumineux 53 confondu avec l'axe 50 du faisceau X ou haute énergie. Le dispositif réalise donc la fonction du télémètre.

Le relevé du contour externe d'une section du patient n'est également qu'un cas particulier correspondant au relevé de plusieurs points pour des positions du faisceau lumineux contenues dans un plan perpendiculaire à l'axe de rotation 35 du bras 34.

Le système selon l'invention a été décrit avec un dispositif de balayage lumineux 37 et un récepteur d'image 38 qui sont montés sur le bras 34 du statif 30. Cependant le système peut être mis en oeuvre avec un dispositif de balayage lumineux et un récepteur d'image qui seraient montés sur un support indépendant de celui qui porte la source de rayons X ou de haute énergie 36 à condition que la position du faisceau lumineux et celle du récepteur d'image soient connues et fournies au système informatique 32. En outre, il faut que le point d'oscillation 54 du faisceau lumineux puisse être mis en coïncidence avec la source de rayons X ou haute énergie pour remplir la fonction du délinéateur.

Dans la description du dispositif de balayage lumineux, on a indiqué que le faisceau lumineux 41 ou 53 était étroit mais il peut avoir la forme d'un faisceau plan. Dans ce cas, la fonction du délinéateur est remplie plus difficilement mais la fonction du conformateur est remplie plus simplement car tous les points du contour extérieur d'une section du patient par le plan du faisceau lumineux fournissent simultanément une image sur la réception d'image. Avec un tel faisceau lumineux, plan, il suffit d'un déflecteur lumineux à un seul axe de rotation pour faire le relevé de plusieurs sections planes.

Le récepteur d'image peut être, par exemple, une caméra de télévision qui fournit une image numérique et qui emploie un tube analyseur d'énergie ou une matrice de photodiodes. On obtient alors une image à deux dimensions.

On peut également utiliser un récepteur d'image qui fournit une image à une seule dimension, le capteur sera alors, par exemple, une barrette linéaire de photodiodes.

L'invention concerne également un procédé pour mesurer et/ou vérifier la position d'un patient par rapport à un faisceau de rayons en vue d'un traitement de radiothérapie. Sur la base de la description faite ci-dessus du système, les opérations sont les suivantes :

a) le balayage par le faisceau lumineux 53 du corps du patient 7 pour obtenir des points d'impact déterminés du faisceau lumineux, ces points d'impact correspondant à la distance source-peau, à une section transversale du patient ou au contour du faisceau de radiothérapie sur le corps du patient;

b) la détection de ces points d'impact par le récepteur image 38 de manière à obtenir une image desdits points;

c) le calcul des coordonnées de ces points d'impact par le dispositif informatique 32 à partir des coordonnées de la source 54 et de celles de l'image desdits points;

d) l'enregistrement des coordonnées de ces points d'impact en relation avec les positions correspondantes du faisceau lumineux qui leur a donné naissance.

Une partie des opérations qui viennent d'être décrites correspondent à celles qui sont effectuées pendant la simulation dans un appareil de radiologie du type diagnostique. Les informations qui en résultent sont utilisées avec celles recueillies à l'aide de l'appareil de radiologie et du tomodensimètre pour définir le plan de traitement du patient. Ce plan définit, outre l'intensité du faisceau de radiothérapie et la durée d'exposition, les positions relatives des différents faisceaux de radiothérapie par rapport au corps du patient et leur contour respectif sur le corps du patient de manière à obtenir des tracés sur le corps du patient. Ces différents tracés sont balayés par le faisceau lumineux 53 en effectuant les opérations a), b), c), et d), décrites ci-dessus, ce qui permet d'obtenir et d'enregistrer les coordonnées des tracés en relation avec les positions correspondantes du faisceau lumineux qui leur a donné naissance. Ce sont ces informations qui sont utilisées dans l'appareil de radiothérapie pour repositionner le corps du patient par rapport aux différents faisceaux selon un processus comparatif qui comprend les opérations suivantes :

e) le balayage du corps du patient par le faisceau lumineux à l'aide des informations de positions du faisceau lumineux qui ont été enregistrées de manière à obtenir les points d'impact dont les coordonnées ont été enregistrées;

f) la mesure de la position réelle de ces points d'impact à l'aide des opérations a), b), c), et d), qui ont été décrites ci-dessus et ainsi obtenir les coordonnées réelles des points d'impact;

g) la comparaison des coordonnées réelles des points d'impacts avec les coordonnées enregistrées de manière à déterminer l'existence ou non d'un écart et,

h) le déplacement du corps du patient pour que les positions réelles coïncident avec les positions enregistrées.

Dans certains cas, il peut être plus simple que les opérations f), g), et h) soient effectuées visuellement par l'opérateur, en comparant le tracé obtenu par un balayage répétitif du faisceau lumineux selon les informations enregistrées de position du faisceau avec le contour marqué sur le corps du patient.

**Revendications**

1. Système de mesure et ou de vérification de la position d'un patient dans un équipement de radiothérapie qui comprend un statif (30) à mouvement isocentrique de centre O, une table (31) sur laquelle est disposé le patient et une source de rayonnement (36) qui peut être à basse énergie pour des opérations de simulation ou à haute énergie pour des opérations de radiothérapie, caractérisé en ce qu'il comprend un dispositif (37) de balayage d'un faisceau lumineux (41, 53) suivant au moins une direction de manière à pouvoir balayer au moins une partie de la surface du corps du patient et un dispositif de commande (32) des positions du faisceau lumineux de manière à déplacer le faisceau lumineux selon un tracé déterminé sur la surface du corps du patient, ledit tracé délimitant la surface d'impact du faisceau de rayons haute énergie.

2. Système selon la revendication 1, caractérisé en ce qu'il comprend en outre un dispositif de détection (38) de l'impact du faisceau lumineux sur le corps du patient et un dispositif de calcul connecté au dispositif de commande (32) de position du faisceau lumineux et au dispositif de détection (38) pour calculer la position de l'impact du faisceau lumineux sur le corps du patient de manière à pouvoir déterminer, d'une part, la distance entre la source de rayons haute énergie et la surface du corps du patient et, d'autre part, le contour extérieur d'une section du corps du patient.

3. Procédé de mesure et/ou de vérification de la position d'un patient dans un équipement de radiothérapie qui comprend un statif (30) à mouvement isocentrique de centre O, une table (31) sur laquelle est disposé le patient et une source de rayonnement (36) qui peut être à basse énergie pour des opérations de diagnostic ou à haute énergie pour des opérations de radiothérapie, ladite source de rayonnement étant portée par le statif (30), caractérisé en ce qu'il comprend les opérations suivantes :

a) un balayage d'au moins une partie de la surface du corps du patient par un faisceau lumineux dont le point de pivotement a une position connue par rapport à la source de rayonnement (36) de manière à obtenir des points d'impact déterminés dudit faisceau sur la surface du corps du patient,

b) une détection de ces points d'impact par l'enregistrement d'une image appropriée,

c) un calcul des coordonées de ces points d'impact à partir de la connaissance de la position de la source et de la position sur l'image de ces points d'impact,

d) un enregistrement des coordonnées de ces points d'impact en relation avec les positions correspondantes du faisceau lumineux qui leur a donné naissance.

4. Procédé selon la revendication 3 caractérisé en ce qu'il comprend en outre les opérations suivantes :

e) un balayage du corps du patient par le faisceau lumineux à l'aide des informations de positions enregistrées du faisceau lumineux pour obtenir les points d'impact dont les coordonnées ont été enregistrées,

f) la mesure de la position réelle de ces points d'impact en mettant en oeuvre les opérations a), b), c), et d).

g) la comparaison des coordonnées réelles des points d'impact avec les coordonnées enregistrées et,

h) le déplacement du corps du patient pour que les positions réelles coïncident avec les positions enregistrées.

FIG_1

FIG_2

FIG_3

# FIG_4

# FIG_5

# DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | PHYSICS IN MEDICINE AND BIOLOGY, vol. 27, no. 2, février 1982, pages 301-305, The Institute of Physics, Bristol, GB; M. ENDO et al.: "Patient beam positioning system using CT images" * En entier * --- | 1,3 | A 61 B 6/08 A 61 N 5/10 |
| A | IDEM --- | 2 | |
| Y | EP-A-0 205 720 (INSTRUMENT AB SCANDITRONIX) * Page 4, ligne 1 - page 8, ligne 14; figures 2,6 * --- | 1 | |
| Y | EP-A-0 062 941 (N.V. PHILIPS' GLOEILAMPENFABRIEKEN) * Figure 1; page 2, ligne 36 - page 6, ligne 1 * | 3 | |
| A | --- | 1,2,4 | |
| A | US-A-4 208 675 (BAJON et al.) * Figure 1; colonne 3, ligne 29 - colonne 6, ligne 3 * ----- | 3 | DOMAINES TECHNIQUES RECHERCHES (Int. Cl.5) A 61 B A 61 N |

Le présent rapport a été établi pour toutes les revendications

| Lieu de la recherche | Date d'achèvement de la recherche | Examinateur |
|---|---|---|
| LA HAYE | 04-12-1989 | CHEN A.H. |